# FASCICULE DE BREVET EUROPEEN

(11) **EP 3 285 774 B1**
(45) Date de publication et mention de la délivrance du brevet: **08.01.2020**
(21) Numéro de dépôt: 16717911.8
(22) Date de dépôt: 20.04.2016
(51) Int. Cl.: A61K 31/5415, A61P 1/16

(54) **UTILISATION DE LA (4-HYDROXY-2-METHYL-1,1-DIOXIDO-2H-BENZO[E][1,2]THIAZIN-3-YL)(NAPHTHALEN-2-YL) METHANONE DANS LA PREVENTION ET/OU LE TRAITEMENT DE LA STEATOHEPATITE NON-ALCOOLIQUE**
VERWENDUNG VON (4-HYDROXY-2-METHYL-1,1-DIOXIDO-2H-BENZO[E] [1,2] THIAZIN-3-YL)(NAPHTHALEN-2-YL)METHANON ZUR VORBEUGUNG UND/ODER BEHANDLUNG VON NICHTALKOHOLISCHER STEATOHEPATITIS
USE OF (4-HYDROXY-2-METHYL-1,1-DIOXIDO-2H-BENZO[E][1,2]THIAZINE-3-YL)(NAPHTHALENE-2-YL) METHANONE IN THE PREVENTION AND/OR TREATMENT OF NON-ALCOHOLIC STEATOHEPATITIS

(30) Priorité: 21.04.2015 FR 1553574
(43) Date de publication de la demande: 28.02.2018
(73) Titulaire: Pierre Fabre Médicament, 92100 Boulogne-Billancourt (FR)
(72) Inventeur: LAMOTHE, Marie, 81100 Castres (FR); JUNQUERO, Didier, 81100 Castres (FR); LE GRAND, Bruno, 81220 Teyssode (FR)
(74) Mandataire: Regimbeau
(86) Numéro de dépôt international: PCT/EP2016/058760
(87) Numéro de publication internationale: WO 2016/169983

(56) Documents cités:
- WO-A1-2010/100139
- WO-A2-02/076435
- STEFAN NORBERT ET AL: "Inhibition of 11[beta]-HSD1 with RO5093151 for non-alcoholic fatty liver disease: a multicentre, randomised, double-blind, placebo-controlled trial", THE LANCET DIABETES & ENDOCRINOLOGY, ELSEVIER, GB, vol. 2, no. 5, 1 mai 2014 (2014-05-01), pages 406-416, XP008178392, ISSN: 2213-8595, DOI: 10.1016/S2213-8587(13)70170-0 [extrait le 2014-02-17]

## Description

La présente invention concerne la (4-hydroxy-2-méthyl-1,1-dioxido-2H-benzo[e][1,2]thiazin-3-yl)(naphthalen-2-yl)méthanone ou l'un de ses sels pharmaceutiquement acceptables pour son utilisation dans la prévention et/ou le traitement de la stéatose hépatique, incluant la stéatohépatite non-alcoolique ou l'une de ses complications.

La (4-hydroxy-2-méthyl-1,1-dioxido-2H-benzo[e][1,2]thiazin-3-yl)(naphthalen-2-yl)méthanone représentée par la formule : 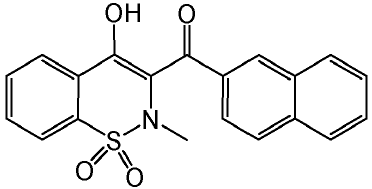 ses sels pharmaceutiquement acceptables ainsi que son utilisation comme traitement tant curatif que préventif du diabète de type 2, de l'obésité, des dyslipidémies, de l'hypertension artérielle, de l'athérosclérose et des pathologies cliniques qui en résultent tels que les accidents coronariens, les accidents vasculaires cérébraux ou l'artérite des membres inférieurs, des hyperglycémies, de l'intolérance au glucose, de la résistance à l'insuline, des hypertriglycéridémies, des hypercholestérolémies, des resténoses, des pancréatites, des rétinopathies, des néphropathies, des neuropathies, de certains types de cancer ou des glaucomes sont décrits dans la demande de brevet WO 2010/100139.

Même si les hépatites virales et la maladie hépatique alcoolique sont cruciales au niveau mondial, elles ne représentent pas toutes les affections hépatiques, même pas les plus importantes. Ces deux dernières décennies, il est apparu de plus en plus évident que la stéatose hépatique non alcoolique (NAFLD) et la stéatohépatite non alcoolique (NASH) représentent actuellement la cause numéro une de maladies hépatiques dans les pays occidentaux.

La stéatose hépatique résulte de l'accumulation de graisses dans les hépatocytes. Les composés graisseux s'accumulent lentement dans le foie lorsque la quantité de graisses accumulée excède celle que son corps peut traiter. Une personne a une stéatose hépatique lorsque la graisse représente au moins 5 % du foie. Une stéatose hépatique simple peut être un état entièrement bénin et n'entraine habituellement pas de lésions au foie. Cependant, une fois qu'il y a accumulation de graisses simples, le foie devient vulnérable à des lésions ultérieures qui peuvent entrainer de l'inflammation et une cicatrisation du foie. La stéatose hépatique devient de plus en plus fréquente chez les enfants en grande partie à cause d'une augmentation alarmante de l'obésité chez les enfants.

La NASH est une pathologie hépatique chronique à forte prévalence, la stéatose étant associée à des lésions histologiques des cellules hépatiques et une inflammation lobulaire (La Brecque et al., World Gastroenterology Organisation Global guidelines, J. Clin. Gastroenterol. 48, 467-473, 2014), éventuellement une fibrose. La NASH est souvent associée à un risque accru de maladies cardiovasculaires et notamment d'accidents vasculaires cérébraux.

La pathogénèse de la NASH est multifactorielle, elle inclut divers mécanismes physiopathologiques conduisant à l'insulino-résistance périphérique et hépatique, aux désordres du métabolisme des acides gras, au stress oxydatif, à la fibroprolifération cellulaire (Cusi, Gastroenterol. 142, 711-725, 2012). Toutefois, à ce jour, la NASH diagnostiquée par une biopsie hépatique n'est pas toujours associée à un diabète ou des dyslipidémies. La complexité et la multimodalité de cette pathologie explique d'une part l'absence de traitement thérapeutique établi et reconnu à ce jour, et d'autre part les essais cliniques en cours et la multiplicité des approches mécanistiques. Toutefois un traitement médical par un dérivé d'acides biliaires présentant des propriétés antioxydantes et anti-inflammatoires peut être envisagé (Coskun et al., Eur. J. Gastroenterol. Hepatol. 27, 142-149, 2015).

Les glucocorticoïdes, le cortisol chez l'homme, sont des hormones ubiquitaires qui jouent un rôle prépondérant dans la régulation du métabolisme énergétique. Ils favorisent la gluconéogenèse, inhibent la sécrétion d'insuline ainsi que la recapture périphérique du glucose. Les 11β-hydroxystéroïde deshydrogénases (11β-HSDs) régulent les niveaux de glucocorticoïdes dans certains tissus cibles, comme le foie, le tissu adipeux, les reins, le cerveau, en assurant l'interconversion de cortisone en cortisol et vice versa (Chapman et al., Physiol. Rev. 93, 1139-1206, 2013 ; Morgan et al., Proc. Natl. Acad. Sci. 111, E2482-E2491, 2014 ; Gathercole et al., Endocrine Rev. 34, 525-555, 2013). Au niveau du tissu adipeux une augmentation locale en cortisol peut conduire à une augmentation exacerbée de la lipolyse qui provoquera une élévation des acides gras libres sanguins et un afflux portal accru de lipides au niveau du foie ; de ce mécanisme nommé « lipotoxicité » résultent une stéatose hépatique, une stimulation de la lipogénèse *de novo* qui peut évoluer vers la NASH, et la néoglucogénèse (Ahmed et al., Plos One 7, e29531, 2012). Au delà de cette interaction « tissu adipeux périphérique - foie », la régénération de cortisol intra-hépatique *via* la 11β-hydroxystéroïde déshydrogénase de type 1 peut également entraîner une néoglucogénèse et accentuer l'insulinorésistance périphérique. Très récemment il a été démontré qu'une réduction de l'activité de la 11β-hydroxystéroïde déshydrogénase de type 1 chez la souris provoque une augmentation des acides biliaires circulants et intra-hépatiques (Penno et al., Mol. Metab. 3, 554-546, 2014) lesquels jouent un rôle clé dans l'homéostasie des lipides et la physiopathologie hépatique (Porez et al., J. Lipid Res. 53, 1723-1737, 2012). Le document WO 02/076435 divulgue le rôle de la 11β-hydroxystéroïde déshydrogénase de type 1 (11β-HSD1) dans l'accumulation de graisse dans le foie et l'utilisation d'inhibiteur de la 11β-HSD1 dans le traitement de la stéatose hépatique, en particulier chez des patients présentant une stéatohépatite non alcoolique.

L'intérêt de l'inhibition de la 11β-hydroxystéroïde déshydrogénase de type 1 a été étudié en clinique dans la stéatose hépatique chez des sujets en surcharge pondérale mais non diabétique (Stefan et al., Lancet Diabetes Endocrinol. 2, 406-416, 2014). Dans cette étude, aucune amélioration de la sensibilité périphérique à l'insuline n'a été rapportée suggérant que cet inhibiteur de la 11β-hydroxystéroïde déshydrogénase de type 1 agit par un autre mécanisme d'action. Néanmoins, avec d'autres composés l'augmentation de la sensibilité à l'insuline a été clairement démontrée (Rosenstock et al., Diabetes Care 33, 1516-1522, 2010) ce qui souligne des différences majeures de profil d'activité au sein d'une même classe thérapeutique et ne permet donc aucune prévisibilité de la distribution tissulaire d'un composé à l'autre.

Ainsi, l'importance d'une inhibition de la 11β-hydroxystéroïde déshydrogénase de type 1 dans tous les tissus cibles métaboliquement actifs et notamment dans le tissu adipeux périphérique en raison de ses interactions avec le foie *via* la lipolyse parait être un facteur clé de succès pour une molécule inhibitrice afin de réduire la lipotoxicité hépatique et traiter la NASH et les pathologies associées.

Les inventeurs ont découvert de façon inattendue que la (4-hydroxy-2-méthyl-1,1-dioxido-2H-benzo[e][1,2]thiazin-3-yl)(naphthalen-2-yl)méthanone ou l'un de ses sels pharmaceutiquement acceptables, en particulier le sel de potassium, pouvait être utilisée comme traitement de la stéatose hépatique, notamment de la NASH ou l'une de ses complications.

Dans la présente invention, le terme « pharmaceutiquement acceptable » se réfère à des entités moléculaires et des compositions qui ne produisent aucun effet adverse, allergique ou autre réaction indésirable quand elles sont administrées à un humain. Quand utilisé ici, le terme « excipient pharmaceutiquement acceptable » inclut tout diluant, adjuvant ou excipient, tels que des agents préservatifs, des agents de remplissage, des agents désintégrants, mouillants, émulsifiants, dispersants, antibactériens ou antifongiques, ou bien encore des agents qui permettraient de retarder l'absorption et la résorption intestinale et digestive. L'utilisation de ces milieux ou vecteurs est bien connu de l'homme du métier.

Les sels pharmaceutiquement acceptables pour l'usage thérapeutique du composé de la présente invention comprennent les sels non toxiques conventionnels du composé de l'invention tels que ceux formés à partir de bases organiques ou inorganiques. A titre d'exemple on peut citer les sels dérivés des bases inorganiques comme la soude, la potasse ou l'hydroxyde de calcium et les sels dérivés de bases organiques comme la lysine ou l'arginine.

Ces sels peuvent être synthétisés à partir du composé de l'invention contenant une partie acide et les bases correspondants selon les méthodes chimiques conventionnelles.

Le proton de la fonction hydroxyle présent dans la (4-hydroxy-2-méthyl-1,1-dioxido-2H-benzo[e][1,2]thiazin-3-yl)(naphthalen-2-yl)méthanone (composé 1) est suffisamment acide pour pouvoir être salifié selon les techniques connues de l'homme du métier. Ainsi à titre d'exemple, le potassium 3-(2-naphthoyl)-2-méthyl-2H-benzo[e] [1,2]thiazin-4-olate 1,1-dioxide, peut être obtenu après dissolution du composé 1 dans un mélange 50/50 d'éthanol et de dichlorométhane puis ajout d'un équivalent d'une solution aqueuse 1M de potasse. La présente invention concerne plus particulièrement ce sel particulier de potassium. Un deuxième exemple est le sodium 3-(2-naphthoyl)-2-méthyl-2H-benzo[e] [1,2]thiazin-4-olate 1,1-dioxide, obtenu comme précédemment en replaçant la solution aqueuse de potasse par une solution aqueuse de soude.

Les solvates acceptables pour l'usage thérapeutique du composé de la présente invention comprennent les solvates conventionnels tels que ceux formés lors de la dernière étape de préparation du composé de l'invention du fait de la présence de solvants. A titre d'exemple on peut citer les solvates dus à la présence d'eau ou d'éthanol.

Selon la présente invention, les complications possibles de la NASH sont : la fibrose hépatique, la cirrhose, l'insuffisance hépatique et le carcinome hépatocellulaire.

La fibrose hépatique est la résultante commune aux maladies chroniques du foie, caractérisée par l'accumulation anormalement élevée de constituants de la matrice extracellulaire dans le parenchyme hépatique. Sa progression peut conduire à la cirrhose.

La cirrhose peut résulter de diverses causes, la consommation chronique d'alcool, d'accumulation de graisses dans le foie, de maladies auto-immunes. Elle est définie selon des critères morphologiques de fibrose et de transformation de l'architecture normale du foie en nodules structurellement anormaux. Ces anomalies s'accompagnent de bouleversements de la fonction hépatique.

L'insuffisance hépatique appelée parfois hépatite fulminante est une altération aigüe grave de la fonction hépatocellulaire. Les premières conséquences sont des troubles majeurs de l'hémostase entrainant un risque hémorragique multi-viscéral. Son pronostic est extrêmement sévère.

Le carcinome hépatocellulaire est un cancer primitif du foie. Il se développe habituellement sur une cirrhose. Pour les patients présentant des facteurs de risque de carcinome hépatocellulaire (cirrhose, NASH) une surveillance accrue des paramètres biologiques et morphologiques est maintenant mise en place afin de détecter la maladie à un stade plus précoce.

La (4-hydroxy-2-méthyl-1,1-dioxido-2H-benzo[e][1,2]thiazin-3-yl)(naphthalen-2-yl)méthanone est un inhibiteur de la 11β-hydroxystéroïde déshydrogénase de type 1.

L'invention a pour objet la (4-hydroxy-2-méthyl-1,1-dioxido-2H-benzo[e][1,2]thiazin-3-yl)(naphthalen-2-yl)méthanone ou l'un de ses sels pharmaceutiquement acceptables, pour son utilisation comme médicament dans la prévention et/ou le traitement de la stéatose hépatique.

L'invention a aussi pour objet la (4-hydroxy-2-méthyl-1,1-dioxido-2H-benzo[e][1,2]thiazin-3-yl)(naphthalen-2-yl)méthanone ou l'un de ses sels pharmaceutiquement acceptables, pour son utilisation comme médicament dans la prévention et/ou le traitement de la stéatohépatite non alcoolique ou l'une de ses complications, comme par exemple la fibrose hépatique, la cirrhose, l'insuffisance hépatique ou le carcinome hépatocellulaire.

Il est important de noter que l'utilisation de la (4-hydroxy-2-méthyl-1,1-dioxido-2H-benzo[e][1,2]thiazin-3-yl)(naphthalen-2-yl)méthanone ou l'un de ses sels pharmaceutiquement acceptables convient également comme médicament dans la prévention et/ou le traitement de la stéatose hépatique ou de la stéatohépatite, résiduelle après sevrage alcoolique.

La présente invention concerne en outre une composition pharmaceutique comprenant la (4-hydroxy-2-méthyl-1,1-dioxido-2H-benzo[e][1,2]thiazin-3-yl) (naphthalen-2-yl)méthanone ou l'un de ses sels pharmaceutiquement acceptables comme principe actif et au moins un excipient pharmaceutiquement acceptable, pour son utilisation comme médicament dans la prévention et/ou le traitement de la stéatose hépatique, notamment chez des patients présentant une stéatohépatite non alcoolique ou l'une de ses complications comme par exemple la fibrose hépatique, la cirrhose, l'insuffisance hépatique ou le carcinome hépatocellulaire.

La composition selon l'invention peut être formulée et/ou administrée avec un ou plusieurs autres agents actifs, tel qu'un agent actif dans le diabète de type 2 et/ou des dyslipidémies.

D'une façon préférée la composition selon l'invention peut être formulée et/ou administrée en combinaison avec des médicaments de l'insulino-résistance, comme les biguanides, par exemple la metformine, les insulino-sécréteurs comme les sulfamides hypoglycémiants, les glinides, les analogues du GLP-1, les gliptines ou les inhibiteurs des alpha-glucosidases.

D'une autre façon préférée la composition selon l'invention peut être formulée et/ou administrée en combinaison avec les statines ou inhibiteurs de l'HMG-CoA réductase, les inhibiteurs de l'absorption intestinale du cholestérol, comme l'ézétimibe, les fibrates, les résines échangeuses d'ions ou l'acide nicotinique.

D'une façon particulièrement préférée, la composition selon l'invention peut être formulée et/ou administrée en combinaison avec un anticorps monoclonal dirigé contre l'enzyme PCSK9, comme par exemple l'alirocumab ou l'évolocumab.

Les compositions pharmaceutiques selon la présente invention peuvent être formulées pour l'administration à l'être humain. Les compositions selon l'invention peuvent être administrées par voie orale, sublinguale, sous-cutanée, intramusculaire, intraveineuse, transdermique, locale ou rectale ou bien encore intra-nasale. Dans ce cas le principe actif peut être administré sous formes unitaires d'administration, en mélange avec des supports pharmaceutiques classiques, aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée ou transdermique, topique, intramusculaire, intraveineuse, intra-nasale ou intraoculaires, les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique, la silice ou des analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec au moins un excipient de formulation et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire, intradermique, sous-cutanée), intra-nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut également être formulé sous forme de microcapsules, éventuellement avec un ou plusieurs supports additifs.

Avantageusement, la composition pharmaceutique selon la présente invention est destinée à une administration par voie orale.

Les dosages de la (4-hydroxy-2-méthyl-1,1-dioxido-2H-benzo[e][1,2]thiazin-3-yl)(naphthalen-2-yl)méthanone ou l'un de ses sels pharmaceutiquement acceptables dans les compositions de l'invention peuvent être ajustés afin d'obtenir une quantité de substance qui est efficace pour obtenir la réponse thérapeutique désirée pour une composition particulière à la méthode d'administration. La dose efficace du composé de l'invention varie en fonction de nombreux paramètres tels que, par exemple, la voie d'administration choisie, le poids, l'âge, le sexe, la nature de la pathologie et la sensibilité de l'individu à traiter. En conséquence, la posologie optimale devra être déterminée par le spécialiste en la matière en fonction des paramètres qu'il juge pertinents. Bien que les doses efficaces puissent varier dans de larges proportions, les doses journalières pourraient s'échelonner entre 1 mg et 2000 mg par 24 heures, et préférentiellement entre 50 et 1000 mg, pour un adulte d'un poids moyen de 70 kg, en une ou plusieurs prises.

Les exemples suivants illustrent l'invention.

### Exemple 1 : Inhibition de la 11β-hydroxystéroïde déshydrogénase de type 1 (11β-HSD 1) humaine, test in vitro sur des adipocytes différenciés humains primaire en culture

L'inhibition de l'enzyme 11β-HSD 1 humaine est évaluée sur des adipocytes humains primaires en culture (Zenbio).

### Protocole :

Après décongélation, la viabilité de pré-adipocytes est vérifiée. Les pré-adipocytes sont ensuite mis en microplaques de 96 puits dans un milieu particulier PM-1 fourni par Zenbio, les plaques sont incubées à 37°C avec 5% de CO₂. Au moins un jour après que les cellules soient en confluence, le milieu PM-1 est remplacé par un milieu DM plus spécifique, également fourni par Zenbio contenant de la xanthine isobutyle-méthyle, de l'insuline, de la dexaméthasone, et un agoniste PPAR. Les cellules vont se différentier en adipocytes après au moins 7 jours. Ensuite les adipocytes matures sont maintenus pendant 4 à 6 jours dans un milieu AM de maintien. Puis en présence de sérum traité par du charbon recouvert de dextran, les cellules sont mises en carence de stéroïdes pendant 48 h, et sont pré-traitées avec les inhibiteurs à tester ou leur véhicule (0,1% DMSO) pendant 1 h à 37 °C avant le pulse à la cortisone. De la cortisone tritiée est ajoutée pendant 4 heures pour atteindre une concentration finale de 20 nM. La concentration de cortisol est quantifiée à partir du surnageant cellulaire grâce à la technologie SPA. Les valeurs d'EC₅₀ sont obtenues avec le logiciel SigmaPlot v.11, équation logistique à 4 paramètres, les valeurs reportées proviennent de 3 expérimentations différentes faites sur 3 donneurs différents.

### Inhibiteurs testés :

Composé 1K : sel de potassium du composé 1, potassium 3-(2-naphthoyl)-2-méthyl-2H-benzo[e][1,2]thiazin-4-olate 1,1-dioxide; Composé 2 : 3'-(4-hydroxy-2-méthyl-1,1-dioxido-2H-benzo[e][1,2]thiazine-3-carbonyl)-[1,1'-biphényl]-4-carbonitrile.

**Résultats**

| | EC₅₀ (nM) | Eₘₐₓ (% d'inhibition à 10 µM) |
|---|---|---|
| Composé 1K | 58 | 97 |
| Composé 2 | ∼10 | 99 |

Les composés 1K et 2 sont de puissants inhibiteurs de la 11β-HSD de type 1 dans les adipocytes humains primaires en culture.

### Exemple 2 : évaluation de la bio-activité plasmatique après une simple administration orale de différents composés inhibiteurs de la 11β-HSD de type 1 chez des souris C57BL/6N (biodisponibilité / puissance/efficacité)

### Protocole

L'évaluation est faite chez des souris mâles non mises à jeun de souche C57BL/6N âgées de 4-6 semaines. Les composés à tester ou le véhicule (méthyle cellulose 0,5% dans l'eau, à 10 ml/kg) sont administrés oralement, n=3 souris par traitement.

Les échantillons de sang sont collectés 1 h et 4 h après l'administration, les tubes sont centrifugés pour obtenir le plasma et congelés à -70°C jusqu'à la bio-analyse. La bio-activité plasmatique (volume final 2%) est analysée en utilisant l'inhibition de la 11β-HSD de type 1 humaine comme système détecteur (technologie SPA) pour chaque composé à chaque dose testée. Le pourcentage d'inhibition (versus les souris traitées par le véhicule) est calculé pour chaque dose.

### Composés testés :

Composé 1 : (4-hydroxy-2-méthyl-1,1-dioxido-2H-benzo[e][1,2]thiazin-3-yl)(naphthalen-2-yl)méthanone;
Composé 1K : sel de potassium du composé 1, potassium 3-(2-naphthoyl)-2-méthyl-2H-benzo[e][1,2]thiazin-4-olate 1,1-dioxide;
Composé 2 : 3'-(4-hydroxy-2-méthyl-1,1-dioxido-2H-benzo[e] [1,2]thiazine-3-carbonyl)-[1,1'-biphényl]-4-carbonitrile, décrit dans le brevet WO 2010/100139 ;
Composé 2K : sel de potassium du composé 2, potassium 3-(4'-cyanobiphényl-4-carbonyl)-2-méthyl-2H-benzo[e][1,2]thiazin-4-olate 1,1-dioxide ;
Composé 3 : composé de référence, (3-(1-adamantyl)-6,7,8,9-tétrahydro-5H-[1,2,4]triazolo[4,3-a]azépine, décrit dans la demande de brevet US2005/0070720.

**Résultats**

| | % d'inhibition à 1 h | | | | | % d'inhibition à 4 h | | | | |
|---|---|---|---|---|---|---|---|---|---|---|
| Composés | Doses (mg/kg) | | | | | Doses (mg/kg) | | | | |
| | 0,63 | 2,5 | 10 | 40 | 80 | 0,63 | 2,5 | 10 | 40 | 80 |
| 1 | 40 | 64 | 81 | 93 | ND | 31 | 60 | 81 | 94 | ND |
| 1K | 47 | 78 | 91 | ND | ND | 36 | 67 | 86 | ND | ND |
| 2 | 47 | 75 | 91 | ND | ND | 39 | 74 | 91 | ND | ND |
| 2K | 26 | 63 | 84 | ND | ND | 29 | 65 | 86 | ND | ND |
| 3 | ND | ND | 93 | 98 | 101 | ND | ND | 22 | 68 | 94 |

| | | | | | | | | | | |
|---|---|---|---|---|---|---|---|---|---|---|
| ND : non déterminé | | | | | | | | | | |

Le profil d'activité du composé 2 est sensiblement supérieur à celui du composé 1. La salification des composés 1 et 2 a des effets opposés quant à l'activité dans ce modèle.

| | | Plasma 1 h | | Plasma 4 h | |
|---|---|---|---|---|---|
| | Dose (mg/kg) | (ng/ml) | % inh. | (ng/ml) | % inh. |
| Composé 1K | 2,5 | 4237 ± 1260 | 75 ± 4 | 3472 ± 2433 | 63 ± 6 |
| | 10 | 13900 ± 2117 | 89 ± 1 | 6813 ± 674 | 85 ± 2 |
| Composé 1 | 2,5 | 2598 ± 1478 | 65 ± 8 | 2205 ± 1250 | 58 ± 5 |
| | 10 | 3675 ± 1781 | 75 ± 8 | 3823 ± 2011 | 73 ± 4 |

| | | | | | |
|---|---|---|---|---|---|
| Inh. : inhibition. | | | | | |

Le composé 1 est détecté dans le plasma des souris traitées et inhibe la 11β-HSD de type 1 dès 0,63 mg/kg. Les analyses LC/MS des échantillons plasmatiques des animaux traités par le composé 1K révèlent un taux plasmatique de la molécule parent largement supérieur à celui trouvé pour le composé 1.

### Exemple 3 : évaluation de la bio-activité du tissu adipeux blanc après une simple administration orale de différents composés inhibiteurs de la 11β-HSD de type 1 chez des souris C57BL/6N (distribution/puissance/efficacité)

### Protocole

Après 4 heures post administration, les souris sont euthanasiées, du tissu adipeux blanc inguinal est prélevé et congelé à -70°C jusqu'au jour des bio-analyses. Le tissu adipeux blanc est homogénéisé dans de l'azote liquide puis traité par de l'acétonitrile (1 ml d'eau distillée pour 4 ml d'acétonitrile et 200 mg de tissu adipeux blanc inguinal) pour extraire les substances solubles. La fraction acétonitrile est récupérée, puis séchée, et le résidu est dissous dans 1 ml de DMSO pour 300 mg de tissu adipeux blanc. La bio-activité du tissu adipeux blanc inguinal (l'équivalent de 150 µg de tissu par puits, 1% DMSO) est analysée en utilisant l'inhibition de la 11β-HSD de type 1 humaine comme système détecteur (technologie SPA) pour chaque composé et pour chaque dose testée.

Composés testés, les mêmes que ceux de l'exemple 2.

**Résultats**

| | % d'inhibition après 4 h | | | | |
|---|---|---|---|---|---|
| Composés | Doses (mg/kg) | | | | |
| | 0,63 | 2,5 | 10 | 40 | 80 |
| 1 | 8 | 36 | 63 | 79 | ND |
| 1K | 21 | 41 | 70 | ND | ND |
| 2 | 24 | 58 | 82 | ND | ND |
| 2K | 17 | 46 | 72 | ND | ND |
| 3 | ND | ND | 15 | 8 | 13 |

| | | | | | |
|---|---|---|---|---|---|
| ND : non déterminé | | | | | |

Comme dans l'exemple 2, le composé 2 est sensiblement plus actif que le composé 1 et la salification conduit a des effets opposés.

### Exemple 4 : inhibition de la conversion de la prednisone en prednisolone après une simple administration des composés 1, 1K et 2K à des singes cynomolgus (puissance/efficacité)

### Protocole

Le protocole utilisé est décrit par Bhat et al. (J. Pharmacol. Exp. Ther. 324, 299-305, 2008). Le primate a été choisi comme espèce, car l'enzyme 11β-HSD montre une spécificité d'espèces très marquée entre le rongeur et l'homme.

Brièvement, l'étude est réalisée chez des singes cynomolgus mâles adultes naïfs mis à jeun toute la nuit. L'inhibiteur de la 11β-HSD de type 1 ou son véhicule (méthylcellulose à 0,5% dans de l'eau) est administré grâce à une intubation naso-gastrique. Deux heures après, un challenge à la prednisone administrée oralement (10 mg/kg) est pratiqué et des échantillons de sang sont régulièrement prélevés pendant 24 heures. Ces échantillons sont centrifugés afin d'obtenir le plasma et sont immédiatement congelés à -70°C jusqu'à la bio-analyse. Les niveaux plasmatiques de prednisone, de prednisolone et des inhibiteurs de la 11β-HSD de type 1 sont mesurés par des méthodes analytiques LC-MS/MS. L'étude est réalisée sur une période de 10 semaines. Les profils de concentration plasmatique en fonction du temps et les aires sous la courbe sont calculés en utilisant la méthode trapézoïdale pour tous les paramètres.

Trois études ont été réalisées :

### - Etude n°1

L'effet du composé 1 est évalué à trois doses, 1,25 ; 5 et 20 mg/kg.

L'inhibition de la 11β-HSD de type 1 médiée par le composé 1 est caractérisée par une réduction de la conversion de la prednisone en prednisolone et reflète un impact hépatique prédominant dans ce délai particulier. Le ratio prednisolone/prednisone plasmatique, de l'aire sous la courbe calculé entre 30 minutes et 4 heures est le paramètre clé pour l'évaluation de l'efficacité. Dans cette première étude, le composé 1 montre une inhibition de la 11β-HSD de type 1 significative et dose-dépendante à partir de 5 mg/kg, l'inhibition atteignant 34% à 20 mg/kg. En considérant la variabilité interindividuelle de cette étude faite chez le singe, 80% des animaux traités par le composé 1 à 20 mg/kg répondent favorablement au traitement pharmacologique avec une inhibition du ratio prednisolone/prednisone de 46%. De la même façon, 60% des animaux répondent favorablement à la dose de 5 mg/kg du composé 1 avec une inhibition atteignant 28%. Il convient de souligner que l'impact sur le tissu adipeux, pour lequel le composé 1 semble avoir une très bonne distribution comme le montre les résultats chez la souris, n'est pas adressé dans ce modèle, dû au délai particulier de l'expérimentation. Aucun signe clinique ou comportemental particulier n'a été rapporté avec ce composé 1 tout au long de cette étude.

### - Etude n° 2

L'effet du composé 1K est évalué à trois doses, 20 ; 40 et 80 mg/kg. Le protocole est tout à fait comparable au précédent, si ce n'est que 5 échantillons de sang par singe étaient prélevés au lieu de 7. Le composé 1K, a également été évalué sur une période de 10 semaines.

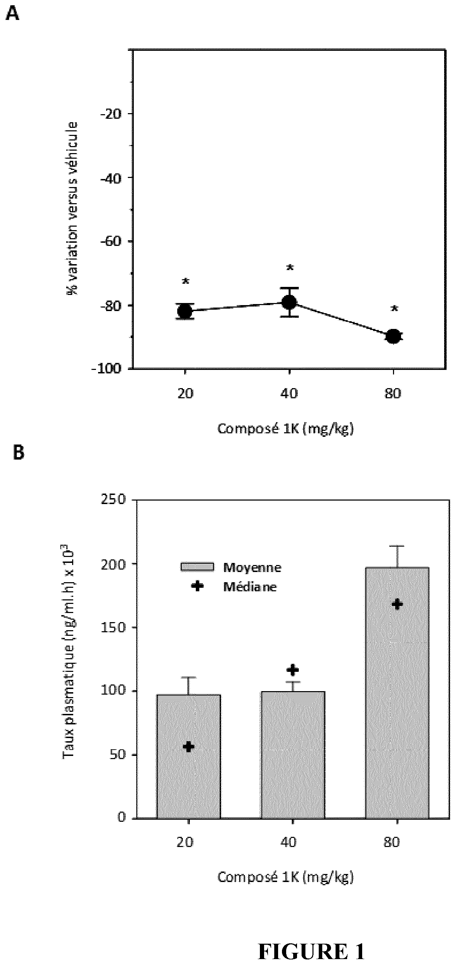

La conversion de prednisone en prednisolone est dépendante de l'activité de la 11β-HSD de type 1, le composé 1K induit une inhibition « maximale » (d'au moins 80%) du ratio prednisolone/prednisone plasmatique, de l'aire sous la courbe calculé entre 30 minutes et 4 heures, et ce résultat est obtenu pour toutes les doses testées (Figure 1A), avec 5 animaux par groupe, la réponse pharmacologique présente une faible variabilité interindividuelle. La figure 1 représente les effets du composé 1K sur le ratio de prednisolone/prednisone plasmatique, calculé avec l'aire sous la courbe entre 30 minutes et 4 heures (panel A) et l'exposition plasmatique pendant la période expérimentale de 6 heures (panel B).

A la même dose de 20 mg/kg, le sel de potassium 1K et la forme non salifiée (composé 1) inhibent ce biomarqueur respectivement par 81 et 34%. Cette différence majeure de l'activité pharmacologique est probablement reliée à l'exposition plasmatique qui augmente près de 8 fois avec le composé 1K. Ce protocole permet d'évaluer principalement l'activité de la 11β-HSD de type 1 hépatique. Aux plus fortes doses 40 et 80 mg/kg, l'exposition plasmatique du composé 1K augmente encore (Figure 1B), et la conversion de prednisone en prednisolone est significativement inhibée par respectivement 79 et 89%. Aucun signe clinique ou comportemental particulier n'a été rapporté avec le composé 1K tout au long de cette étude, même à 80 mg/kg.

### - Etude n°3

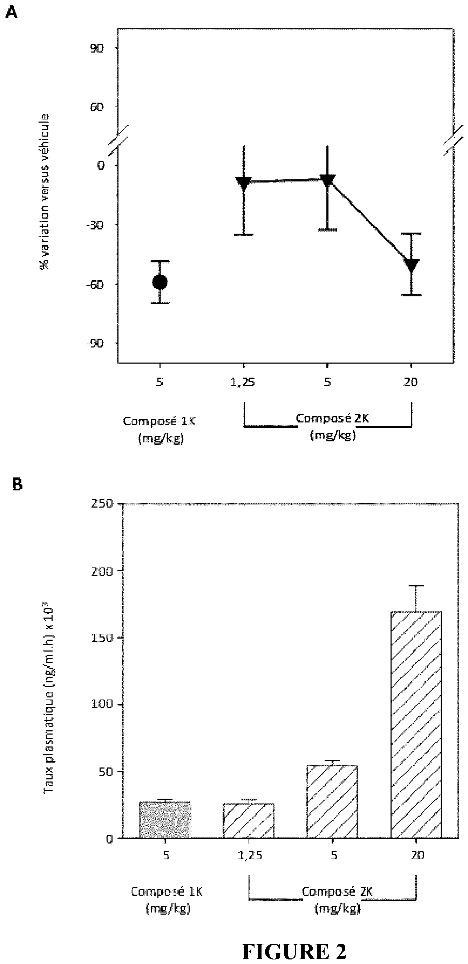

Un protocole expérimental comparable aux précédents, sur cinq primates naïfs a été réalisé pour tester une dose-réponse (1,25 ; 5 et 20 mg/kg) du composé 2K, et pour comparaison une dose de 1K (5 mg/kg). La conversion de prednisone en prednisolone qui dépend de l'activité de la 11β-HSD de type 1 a été évaluée. Le composé 2K inhibe d'environ 50% cette activité à la dose de 20 mg/kg (Figure 2A). La figure 2 représente les effets du composé 2K aux 3 doses testées, sur le ratio de prednisolone/prednisone plasmatique, calculé avec l'aire sous la courbe entre 30 minutes et 4 heures (panel A) et l'exposition plasmatique pendant la période expérimentale de 6 heures (panel B), les résultats obtenus avec le composé 1K à la dose de 5 mg/kg sont comparés sur les 2 panels. Il ressort de cette étude que le composé 2K aux doses inférieures à 20 mg/kg est inactif, il est de façon surprenante moins puissant que le composé 1K alors même que son exposition plasmatique est supérieure (Figure 2B). Ce profil d'activité beaucoup moins favorable pour le composé 2K, n'est pourtant pas dû à une puissance intrinsèque d'inhibition de la 11β-HSD de type 1 inférieure que le composé 1K, bien au contraire (voir exemple 1).

### Exemple 5 : inhibition de la 11β-HSD de type 1 hépatique après une simple administration orale du composé 1K chez le singe cynomolgus (activité ex vivo)

### Protocole

Cinq singes cynomolgus adultes mâles (les mêmes animaux que ceux utilisés dans les études 1 et 2 de l'exemple 4) sont mis à jeun la veille au soir de l'expérimentation. Des prélèvements sanguins sont faits juste avant l'administration du composé 1K (dose de 20 mg/kg, n=3) ou l'administration du véhicule (méthyle cellulose à 0,5% dans l'eau, n=2) puis 4 heures après l'administration orale. Les animaux sont euthanasiés, une nécropsie est réalisée sur les singes, des échantillons de plasma et de tissus cibles sont prélevés puis congelés dans l'azote liquide et stockés à - 70°C jusqu'aux analyses *ex vivo.* Pour tester l'activité *ex vivo* de la 11β-HSD de type 1, des échantillons de 5-10 mg de foie étaient incubés en duplicate pendant 30 minutes à 37°C avec 17 nM [1,2-³H] cortisone dans 50 mM HEPES (pH 7,4) / 100 mM KCl/5 mM NaCl/2 mM MgCl₂. Les taux plasmatiques ont été mesurés par la méthode analytique LC-MS/MS. Les échantillons de foie étaient homogénéisés par sonication et extrait par l'acétonitrile (1ml d'eau distillée, 4 ml d'acétonitrile pour 200 mg de tissu). Les taux tissulaires des composés ont été mesurés par la méthode analytique LC-MS/MS.

### Résultats

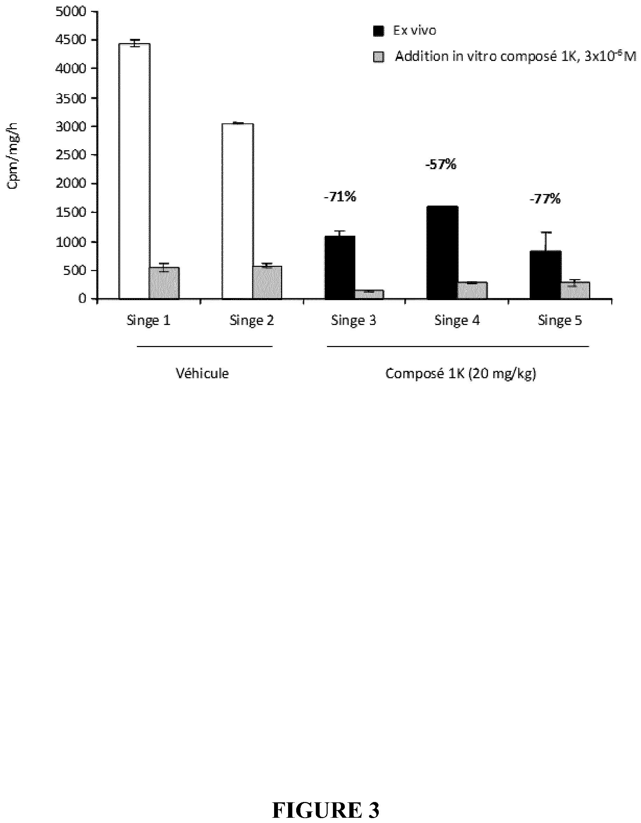

Quatre heures après l'administration orale, les taux plasmatiques moyennés du composé 1K étaient de 16967 ± 3576 ng/ml (SEM), ces données sont en parfaites adéquation avec les résultats des études précédentes. La figure 3 montre les effets de l'administration du composé 1K à 20 mg/kg sur l'activité *ex vivo* de la 11β-HSD de type 1 dans le foie de singes cynomolgus.

Une seule administration orale du composé résulte en une inhibition conséquente de 68% de l'activité ex vivo de la 11β-HSD de type 1 hépatique, laquelle peut être encore davantage inhibée par l'ajout *in vitro* de 3x10⁻⁶ M de composé 1K (Figure 3). Les niveaux hépatiques moyennés (en valeur relative) du composé 1K sont 13,6 ± 3,7 ng/mg de tissu.

### Exemple 6 : inhibition de la 11β-HSD de type 1 du tissu adipeux (mésentérique et sous-cutané) après une simple administration orale du composé 1K à des singes cynomolgus (activité ex vivo)

### Protocole

Il s'agit du même protocole que celui décrit dans l'exemple 5. Mis à part que les échantillons de tissus provenaient du tissu adipeux mésentérique, sous-cutané et inguinal.

### Résultats

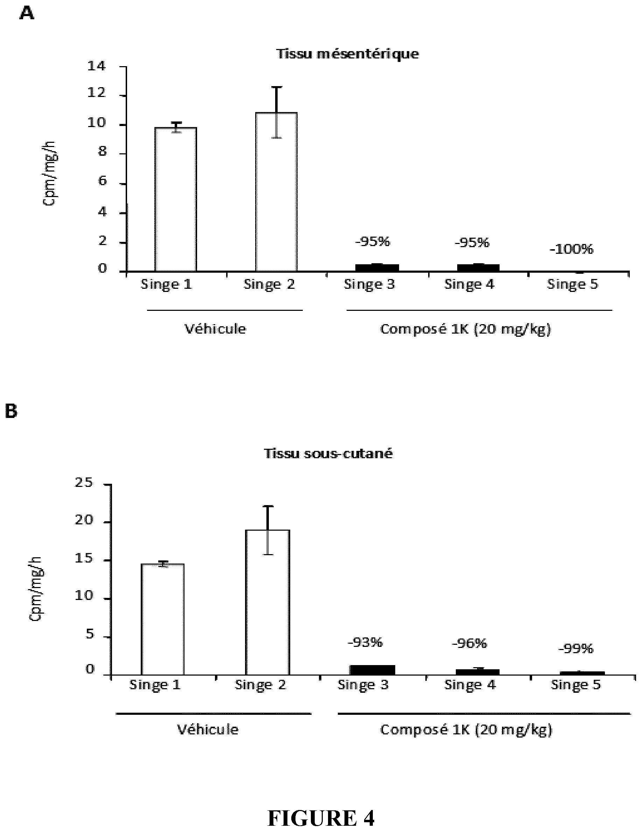

Sur les figures 4 sont résumés les effets de l'administration du composé 1 à 20 mg/kg sur l'activité *ex vivo* de la 11β-HSD de type 1 dans le tissu adipeux de singes cynomolgus. Une simple administration orale du composé 1K induit une inhibition complète de l'activité *ex vivo* de la 11β-HSD de type 1 du tissu adipeux périphérique et viscéral. Les résultats sur le tissu adipeux inguinal sont comparables (données non montrées). Les niveaux tissulaires (en valeur relative) du composé 1K dans les tissus adipeux mésentérique, sous-cutané et inguinal sont respectivement de 6,6 ± 0,4 ; 1,3 ± 0,1 et 6,1 ± 1,5 ng/mg.

Ces résultats démontrent le tropisme extrêmement important du composé 1K pour pénétrer dans le tissu adipeux.

## Revendications

1. La (4-hydroxy-2-méthyl-1,1-dioxido-2H-benzo[e][1,2]thiazin-3-yl)(naphthalen-2-yl)méthanone ou l'un de ses sels pharmaceutiquement acceptables pour son utilisation comme médicament pour la prévention et/ou le traitement de la stéatose hépatique.

2. La (4-hydroxy-2-méthyl-1,1-dioxido-2H-benzo[e][1,2]thiazin-3-yl)(naphthalen-2-yl)méthanone pour son utilisation selon la revendication 1, sous la forme de son sel de potassium.

3. La (4-hydroxy-2-méthyl-1,1-dioxido-2H-benzo[e][1,2]thiazin-3-yl)(naphthalen-2-yl)méthanone ou l'un de ses sels pharmaceutiquement acceptables pour son utilisation selon la revendication 1 ou 2, chez des patients présentant une stéatohépatite non alcoolique.

4. La (4-hydroxy-2-méthyl-1,1-dioxido-2H-benzo[e][1,2]thiazin-3-yl)(naphthalen-2-yl)méthanone ou l'un de ses sels pharmaceutiquement acceptables pour son utilisation selon l'une des revendications 1 à 3, chez des patients présentant une fibrose hépatique, une cirrhose, une insuffisance hépatique ou un carcinome hépatocellulaire.

5. Composition pharmaceutique **caractérisée en ce qu'**elle contient comme principe actif la (4-hydroxy-2-méthyl-1,1-dioxido-2H-benzo[e][1,2]thiazin-3-yl)(naphthalen-2-yl)méthanone ou l'un de ses sels pharmaceutiquement acceptables, en particulier sous la forme de son sel de potassium et au moins un excipient pharmaceutiquement acceptable, pour son utilisation comme médicament pour la prévention et/ou le traitement de la stéatose hépatique.

6. Composition pharmaceutique pour son utilisation selon la revendication 5, chez des patients présentant une stéatohépatite non alcoolique.

7. Composition pharmaceutique pour son utilisation selon l'une des revendications 5 ou 6, chez des patients soumis à un traitement du diabète de type 2 ou de dyslipidémies.

## Patentansprüche

1. (4-Hydroxy-2-methyl-1,1-dioxido-2H-benzo[e][1,2]thiazin-3-yl)(naphthalen-2-yl)methanon oder eines seiner pharmazeutisch annehmbaren Salze für seine Verwendung als Arzneimittel zur Vorbeugung und/oder Behandlung von Steatohepatitis.

2. (4-Hydroxy-2-methyl-1,1-dioxido-2H-benzo[e][1,2]thiazin-3-yl)(naphthalen-2-yl)methanon für seine Verwendung nach Anspruch 1, in Form seines Kaliumsalzes.

3. (4-Hydroxy-2-methyl-1,1-dioxido-2H-benzo[e][1,2]thiazin-3-yl)(naphthalen-2-yl)methanon oder eines seiner pharmazeutisch annehmbaren Salze für seine Verwendung nach Anspruch 1 oder 2 bei Patienten, die eine nichtalkoholische Steatohepatitis aufweisen.

4. (4-Hydroxy-2-methyl-1,1-dioxido-2H-benzo[e][1,2]thiazin-3-yl)(naphthalen-2-yl)methanon oder eines seiner pharmazeutisch annehmbaren Salze für seine Verwendung nach einem der Ansprüche 1 bis 3 bei Patienten, die eine Leberfibrose, eine Zirrhose, ein Leberversagen oder ein Leberzellkarzinom aufweisen.

5. Pharmazeutische Zusammensetzung, **dadurch gekennzeichnet, dass** sie als Wirkstoff (4-Hydroxy-2-methyl-1,1-dioxido-2H-benzo[e][1,2]thiazin-3-yl)(naphthalen-2-yl)methanon oder eines seiner pharmazeutisch annehmbaren Salze enthält, insbesondere in Form seines Kaliumsalzes, und mindestens einen pharmazeutisch annehmbaren Trägerstoff für ihre Verwendung als Arzneimittel zur Vorbeugung und/oder Behandlung von Steatohepatitis.

6. Pharmazeutische Zusammensetzung für ihre Verwendung nach Anspruch 5 bei Patienten, die eine nichtalkoholische Steatohepatitis aufweisen.

7. Pharmazeutische Zusammensetzung für ihre Verwendung nach einem der Ansprüche 5 oder 6, bei Patienten, die einer Behandlung von Typ-2-Diabetes oder von Dyslipidämien unterzogen sind.

## Claims

1. (4-Hydroxy-2-methyl-1,1-dioxido-2H-benzo[e][1,2]thiazin-3-yl)(naphthalen-2-yl)methanone, or a pharmaceutically acceptable salt thereof, for use as a medicinal product for the prevention and/or treatment of fatty liver.

2. (4-Hydroxy-2-methyl-1,1-dioxido-2H-benzo[e][1,2]thiazin-3-yl)(naphthalen-2-yl)methanone for use according to claim 1, in the form of the potassium salt thereof.

3. (4-Hydroxy-2-methyl-1,1-dioxido-2H-benzo[e][1,2]thiazin-3-yl)(naphthalen-2-yl)methanone, or a pharmaceutically acceptable salt thereof, for use according to claim 1 or 2, in patients with non-alcoholic steatohepatitis.

4. (4-Hydroxy-2-methyl-1,1-dioxido-2H-benzo[e][1,2]thiazin-3-yl)(naphthalen-2-yl)methanone, or a pharmaceutically acceptable salt thereof, for use according to one of claims 1 to 3, in patients with liver fibrosis, cirrhosis, liver failure or hepatocellular carcinoma.

5. Pharmaceutical composition **characterized in that** it contains as active ingredient (4-hydroxy-2-methyl-1,1-dioxido-2H-benzo[e][1,2]thiazin-3-yl)(naphthalen-2-yl)methanone, or a pharmaceutically acceptable salt thereof, in particular in the form of the potassium salt thereof, and at least one pharmaceutically acceptable excipient, for use as a medicinal product for the prevention and/or treatment of fatty liver.

6. Pharmaceutical composition for use according to claim 5, in patients with non-alcoholic steatohepatitis.

7. Pharmaceutical composition for use according to one of claims 5 or 6, in patients undergoing treatment for type 2 diabetes or dyslipidemia.
